# EUROPEAN PATENT APPLICATION

(11) **EP 3 238 713 A1**
(43) Date of publication of application: **01.11.2017**
(21) Application number: 17168457.4
(22) Date of filing: 27.04.2017
(51) Int. Cl.: A61K 9/20, A61K 9/24, A61K 31/135, A61K 31/192

(54) **PHARMACEUTICAL COMPOSITIONS OF FLURBIPROFEN AND TRAMADOL**

(30) Priority: 28.04.2016 TR 201605504
(71) Applicant: Sanovel Ilac Sanayi ve Ticaret A.S., 34460 Istanbul (TR)
(72) Inventor: TÜRKYILMAZ, Ali, 34460 Istanbul (TR); DURGUN, Deniz Ömür, 34460 Istanbul (TR); PEHLIVAN AKALIN, Nur, 34460 Istanbul (TR); HATIRNAZ, Zekiye Basak, 34460 Istanbul (TR)
(74) Representative: Sevinç, Erkan

(57) **Abstract**

The present invention relates to a pharmaceutical composition comprising flurbiprofen and tramadol or pharmaceutically acceptable salts thereof and at least one pharmaceutically acceptable excipient.

## Description

### Field of the Invention

The present invention relates to a pharmaceutical composition comprising flurbiprofen and tramadol or a pharmaceutically acceptable salts thereof and at least one pharmaceutically acceptable excipient.

### Background of the Invention

Flurbiprofen is a propionic acid derivative which is an NSAID (non-steroidal anti-inflammatory drug), having analgesic and anti-inflammatory activities. Its chemical structure is illustrated with Formula 1 given below.

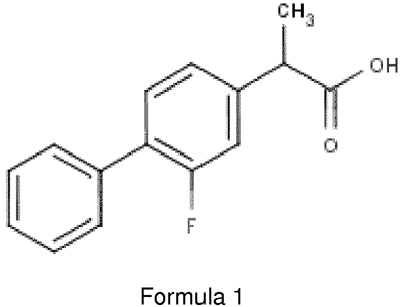

Flurbiprofen is used for alleviating pain in muscle-skeleton system and joint disorders such as ankylosing spondylitis, osteoarthritis, and rheumatoid arthritis, in soft tissue injuries such as sprains and strains, in postoperative cases, and in painful severe menstruation and migraine. It is in the market under the brandname of ANSAID^{®} in strength of 50, 100, 200 and 300 mg. It is recommended 2, 3 or 4 times a day dose.

Another molecule that is used as an analgesic is tramadol disclosed in the patent US6339105 (B1). Tramadol is a centrally acting synthetic opioid analgesic. The chemical name for tramadol hydrochloride is (±)cis-2-[(dimethylamino)methyl]-1-(3methoxyphenyl) cyclohexanol hydrochloride. Although its mode of action is not completely understood, from animal tests, at least two complementary mechanisms appear applicable: binding of parent and M1 metabolite to µ-opioid receptors and weak inhibition of re-uptake of norepinephrine and serotonin.

Many challenges also occur such as (a) the physicochemical compatibility between the different active agents and/or between the active agents and the excipients used; and (b) the therapeutical compatibility between the two active agents regarding their pharmacokinetic and/or pharmaceutical properties in order that the posology of the combined formulation allows to obtain safe and efficient plasma levels of both pharmacological agents.

Use of flurbiprofen and tramadol in combination may cause some, particularly gastrointestinal, side effects. Flurbiprofen shows burning sensation in the gastrointestinal system and tramadol shows nausea, vomiting, dizziness, dry mouth, and sedation. Moreover, the use of tramadol with flurbiprofen can cause additional effects. Avoiding these systemic adverse effects of flurbiprofen and tramadol is very important for the patient.

Instead of one per se use, combining more than one molecule in one dosage form increases the patients' quality of life and patients' compliance. In literature, the combination of flurbiprofen and tramadol has been studied. However, there is no study on combination of flurbiprofen and tramadol in a same dosage form.

Considering all these, combinations of flurbiprofen and tramadol in a suitable pharmaceutical dosage formulation, is needed. In this present invention, a composition has been formulated as to combine flurbiprofen and tramadol in the same dosage form.

### Description of the Invention

The present invention relates to a pharmaceutical composition comprising flurbiprofen and tramadol or a pharmaceutically acceptable salts thereof and at least one pharmaceutically acceptable excipient.

In this embodiment of this present invention, a fixed dose combination comprising flurbiprofen and tramadol has been developed for the management of moderately severe pain in adults. An improved analgesic effect has been achieved with the synergistic effect of flurbiprofen and tramadol.

In one embodiment, in this present invention, pharmaceutical combination formulated in one dosage form providing desired dissolution profile for both flurbiprofen and tramadol.

According to one embodiment, the pharmaceutical acceptable salt of tramadol is tramadol HCl.

Using different drugs may induce incompatibility problems and undesired dissolution profiles that cause undesired side effects. In this present invention, a pharmaceutical dosage form comprising flurbiprofen in combination with tramadol has been developed with safe and effective dissolution profiles for each drug molecule.

According to one embodiment, flurbiprofen is present in an amount of between 10mg and 300mg, preferably between 10mg and 200mg and more preferably it is in an amount of between 50mg and 200mg.

According to one embodiment, tramadol HCl is present in an amount of between 10mg and 200mg, preferably between 20mg and 100mg and more preferably it is in an amount of between 35mg and 100mg.

According to one embodiment, the ratio of flurbiprofen to tramadol HCl is in the range of 0.1 to 30 (w/w), preferably 1 to 15 (w/w), more preferably 1 to 3 (w/w).

According to one embodiment, the ratios used in this present invention ensure the required effective doses for the treatment and desired dissolution profile for both flurbiprofen and tramadol HCl.

An embodiment of this present invention is to combine flurbiprofen and tramadol HCl in a same and stable dosage form with desired dissolution profiles. Pharmaceutical composition of this present invention comprising flurbiprofen in combination with tramadol HCl is in the form of tablet, bilayer tablet, trilayer tablet, multilayer tablet, capsule, tablet in tablet, an inlay tablet, injectable preparate, suspension, syrup, sachet, ointment, cream or gel.

In one embodiment, pharmaceutical composition of this present invention comprising flurbiprofen in combination with tramadol HCl is in the form of a tablet or a bilayer tablet or a trilayer tablet.

In one embodiment, pharmaceutical composition of this present invention comprising flurbiprofen in combination with tramadol HCl is in the form of a tablet.

In one embodiment, pharmaceutical composition of this present invention comprising flurbiprofen in combination with tramadol HCl is in the form of a bilayer tablet.

In one embodiment, pharmaceutical composition of this present invention comprising flurbiprofen in combination with tramadol HCl is in the form of a trilayer tablet.

According to this embodiment, the pharmaceutical composition of this present invention comprises at least one pharmaceutically acceptable excipient.

According to this embodiment, at least one pharmaceutically acceptable excipient is selected from a group comprising diluents, disintegrants, binders, lubricants and glidants or mixtures thereof.

In order to combine two different molecules in one dosage form, molecules should be compatible with each other to achieve desired stability and dissolution for the patients' compliance. During the development study to combine flurbiprofen and tramadol HCl, it has been found that tramadol shows stability problems. The analysis results of combination in conventional tablets shows an increase in impurity results. Owing to the incompatibility problem of these active agents, in this invention formulations have been developed and desired stability has been achieved.

According to one embodiment, due to lactose used as a diluent, stability of both flurbiprofen and tramadol HCl has been achieved. With the synergistic effect of lactose used in the formulation, desired dissolution has also been achieved for both active agents.

According to this embodiment, in this formulation of this present invention, diluent is lactose. The amount of lactose is between 1.0 - 90.00 %, preferably 5.00 - 60.0 % and more preferably it is 5.00 - 30.00 % by weight of total formulation.

According to this embodiment, the amount of lactose in flurbiprofen layer is between 5.00 - 50.00 % by weight of flurbiprofen layer and preferably it is 5.00 - 20.0 % by weight of flurbiprofen layer. The amount of lactose in tramadol layer is between 20.00 - 80.00 % by weight of tramadol layer and preferably it is 20.00 - 40.0 % by weight of tramadol layer.

Suitable disintegrants may include but not limited to sodium starch glycolate, microcrystalline cellulose, croscarmellose sodium, sodium carboxymethyl starch, soy polysaccharide, cross-linked alginic acid, crospovidone, copovidone, gellan gum, xanthan gum, calcium silicate or ion exchange resins or mixtures thereof.

Suitable binders are selected from microcrystalline cellulose (PH 101, PH 102), polyvinylpyrrolidone, crospovidon, sugars, glycose syrups, natural gums, guar gum, gelatins, pullulan, agar, alginate, sodium alginates, K-Caragennen, glycyrrhizin, polymetacrylates, collagen, agar, hyaluronic acid, pectin, tragacanthi gum, carboxymethyl cellulose, polyethylene glycol, polyvinyl alcohol, polyvinyl acetate and their copolymers, cellulose derivatives such as hydroxypropyl methyl cellulose, carboxy methyl cellulose, methyl cellulose, polyvinylalcohol, carrageenan, carbomer, poloxamer, polyacrylamide, aluminum hydroxide, benthonite, laponite, setostearyl alcohol, polyoxyethylene-alkyl ethers, acacia mucilage, polydextrose, polyethylene oxide, xylitol, sucrose stearate, or mixtures thereof.

Suitable lubricants are selected from sodium stearyl fumarate, magnesium stearate, polyethylene glycol, sodium lauryl sulphate, magnesium lauryl sulphate, fumaric acid, glyceryl palmitostearate, hydrogenated natural oils, zinc stearate, calcium stearate, silica, talc, stearic acid, polyethylene glycol, paraffin or mixtures thereof.

Suitable glidants are selected from colloidal silicon dioxide, aluminium silicate or mixtures thereof.

According to one embodiment, the pharmaceutical composition is in the form of a tablet comprising;
a) 10.0 - 50.0 % flurbiprofen,
b) 5.0 - 30.0 % tramadol HCl,
c) 1.0 - 90.0 % lactose,
d) 5.0 - 45.0 % microcrystalline cellulose,
e) 0.5 - 15.0 % hydroxypropyl-cellulose
f) 0.5 - 15.0 % - croscarmellose sodium,
g) 0.1 - 5.0 % colloidal silicon dioxide,
h) 0.1 - 5.0 % - magnesium stearate by weight of total formulation.

According to one embodiment, the pharmaceutical composition is in the form of a bilayer tablet comprising;
- flurbiprofen layer:
   a) 10.0 - 50.0 % flurbiprofen,
   b) 1.0 - 90.0 % lactose,
   c) 5.0 - 45.0 % microcrystalline cellulose,
   d) 0.5 - 15.0 % croscarmellose sodium,
   e) 0.5 - 15.0 % hydroxypropyl cellulose,
   f) 0.3 - 5.0% colloidal silicon dioxide,
   g) 0.1 - 5.0 % magnesium stearate
- tramadol HCl layer:
   h) 5.0 - 30.0 % tramadol HCl,
   i) 8.0 - 45.0 % microcrystalline cellulose,
   j) 1.0 - 90.0 % lactose,
   k) 0.5 - 15.0 % sodium starch glycolate,
   l) 0.5 - 15.0 % hydroxypropylcellulose,
   m) 0.1 - 5.0 % colloidal silicon dioxide,
   n) 0.1 - 5.0 % magnesium stearate by weight of total formulation.

According to one embodiment, the pharmaceutical composition is in the form of a trilayer tablet comprising;
- flurbiprofen layer:
   a) 10.0 - 50.0 % flurbiprofen,
   b) 1.0 - 90.0 % lactose,
   c) 8.0 - 45.0 % microcrystalline cellulose,
   d) 0.5 - 15.0 % croscarmellose sodium,
   e) 0.5 - 15.0 % hydroxypropyl cellulose,
   f) 0.3 - 5.0 % colloidal silicon dioxide,
   g) 0.1 - 5.0 % magnesium stearate
- interlayer:
   h) 0.10 - 5.0 % magnesium starate,
   i) 8.0 - 90.0 % microcrystalline cellulose,
   j) 0.5 - 20 % hydroxypropyl cellulose,
   k) 0.01 - 1.0 % iron oxide yellow.
   l) 0.3 - 5.0 % colloidal silicon dioxide,
- tramadol HCl layer:
   m) 5.0 - 30.0 % tramadol HCl,
   n) 1.0 - 90.0 % lactose,
   o) 0.5 - 15.0 % sodium starch glycolate,
   p) 0.5 - 15.0 % hydroxypropylcellulose,
   q) 0.1 - 5.0 % colloidal silicon dioxide,
   r) 0.1 - 5.0 % magnesium stearate by weight of total formulation.

### Example 1: Tablet

| **ingredient** | **amount %** |
|---|---|
| Flurbiprofen | 10.0 - 50.0 % |
| Tramadol HCl | 5.0 - 30.0 % |
| Lactose monohydrate | 5.0 - 60.0 % |
| Microcrystalline cellulose | 5.0 - 45.0 % |
| Croscarmellose sodium | 0.5 - 20.0 % |
| Hydroxypropyl-cellulose | 0.5 - 20.0 % |
| Colloidal silicon dioxide | 0.1 - 5.0 % |
| Magnesium stearate | 0.1 - 5.0 % |

The process of the formulations is carried out as follows: Flurbiprofen, tramadol HCl, lactose monohydrate and microcrystalline cellulose are taken into fluid bed drier and mixed. The solution of hydroxypropyl-cellulose is sprayed to the mixture and granulation is performed. Granules are dried and sieved. Colloidal silicon dioxide is added to dry granules and mixed. Magnesium stearate is added to this mixture and mixed again. Mixture is pressed into tablets. Tablets are optionally coated with Opadry Pink (31F24886).

### Example 2: Bilaver Tablet

| **ingredient** | **amount %** |
|---|---|
| **Flurbiprofen layer** | |
| Flurbiprofen | 10 - 50.0 % |
| Lactose | 5.0 - 60.0 % |
| Microcrystalline cellulose | 8.0 - 45.0 % |
| Croscarmellose sodium | 0.5 - 20.0 % |
| Hydroxypropyl cellulose | 0.5 - 20.0 % |
| Colloidal silicon dioxide | 0.1 - 5.0 % |
| Magnesium stearate | 0.1 - 10.0 % |

| **Tramadol layer** | |
|---|---|
| Tramadol HCl | 0.5 - 10.0 % |
| Lactose monohydrate | 5.0 - 60.0 % |
| Microcrystalline cellulose | 8.0 - 45.0 % |
| Hydroxypropylmethylcellulose | 0.5 - 20.0 % |
| Colloidal silicon dioxide | 0.1 - 5.0 % |
| Magnesium stearate | 0.1 - 10.0 % |

The process of the formulations is carried out as follows:

### Flurbiprofen layer:

Flurbiprofen, lactose are mixed and sieved. A part of microcrystalline cellulose and colloidal silicon dioxide are sieved and added to mixture. Other part of microcrystalline cellulose -, croscarmellose sodium and hydroxypropyl cellulose are added to this mixture and mixed again. Then, magnesium stearate is added to this mixture and mixed.

### Tramadol layer:

Tramadol HCl, lactose monohydrate, microcrystalline cellulose, lactose monohydrate and hydroxypropylmethylcellulose are taken into collete and powder mixture is granulated with water. The granules are sieved and dried then sieved again. Colloidal silicon dioxide is added to the granules and mixed. Then, magnesium stearate is added and mixed again.

These two mixtures for each layer are pressed into bilayer tablets.

### Example 3: Trilayer Tablet

| **ingredient** | **amount %** |
|---|---|
| **Flurbiprofen layer** | |
| Flurbiprofen | 10 - 50.0 % |
| Lactose monohydrate | 5.0 - 60.0 % |
| Microcrystalline cellulose | 5.0 - 45.0 % |
| Croscarmellose sodium | 0.5-15.0 % |
| Hydroxypropylmethylcellulose | 0.5-15.0 % |
| Colloidal silicon dioxide | 0.1 - 5.0 % |
| Magnesium stearate | 0.1 - 5.0 % |

| **Interlayer** | |
|---|---|
| Magnesium stearate | 0.1 - 10.0 % |
| Microcrystalline cellulose | 5.0 - 45.0 % |
| Hydroxypropylcellulose | 0.2 - 15.0 % |
| Iron oxide yellow | 0.01 - 1.0 % |
| Colloidal silicon dioxide | 0.1 - 5.0 % |

| **Tramadol layer** | |
|---|---|
| Tramadol HCl | 0.5 - 10.0 % |
| Lactose monohydrate | 5.0 - 60.0 % |
| Microcrystalline cellulose | 5.0 - 45.0 % |
| Sodium starch glycolate | 1.0-15.0 % |
| Hydroxypropylmethylcellulose | 0.5-15.0 % |
| Colloidal silicon dioxide | 0.1 - 5.0 % |
| Magnesium stearate | 0.1 - 5.0 % |

The process of the formulations is carried out as follows:

### Flurbiprofen layer:

Flurbiprofen and lactose are mixed and sieved. A part of microcrystalline cellulose and colloidal silicon dioxide are sieved and added to mixture. Other part of microcrystalline cellulose, croscarmellose sodium and hydroxypropyl cellulose are added to this mixture and mixed again. Then, magnesium stearate is added to this mixture and mixed.

### Inert layer:

A part of microcrystalline cellulose and hydroxypropyl cellulose are mixed. Then, iron oxide yellow, colloidal silicon dioxide and other part of microcrystalline cellulose are sieved and added to this mixture. Powder mixture is mixed. Magnesium stearate is added to this mixture and mixed again.

### Tramadol layer:

Tramadol HCl, lactose monohydrate, microcrystalline cellulose, sodium starch glycolate and hydroxypropylmethylcellulose are taken into collete and powder mixture is granulated with water. The granules are sieved and dried then sieved again. Colloidal silicon dioxide is added to the granules and mixed. Then, magnesium stearate is added and mixed again.

These three mixtures for each layer are pressed into trilayer tablets.

## Claims

1. A pharmaceutical composition comprising flurbiprofen and tramadol or a pharmaceutically acceptable salts thereof and at least one pharmaceutically acceptable excipient.

2. The pharmaceutical composition according to claim 1, wherein the pharmaceutical acceptable salt of tramadol is tramadol HCl.

3. The pharmaceutical composition according to claim 1, flurbiprofen is present in an amount of between 10mg and 300mg, preferably between 10mg and 200mg and more preferably it is in an amount of between 50mg and 200mg.

4. The pharmaceutical composition according to claim 2, tramadol HCl is present in an amount of between 10mg and 200mg, preferably between 20mg and 100mg and more preferably it is in an amount of between 35mg and 100mg.

5. The pharmaceutical composition according to claim 1 or 2, wherein the ratio of flurbiprofen to tramadol HCl is in the range of 0.1 to 30 (w/w), preferably 1 to 15 (w/w), more preferably 1 to 3 (w/w).

6. The pharmaceutical composition according to claim 1, wherein said pharmaceutical composition is in the form of tablet, bilayer tablet, trilayer tablet, multilayer tablet, capsule, tablet in tablet, an inlay tablet, injectable preparate, suspension, syrup, sachet, ointment, cream or gel.

7. The pharmaceutical composition according to claim 6, wherein said pharmaceutical composition is in the form of a tablet or a bilayer tablet or a trilayer tablet.

8. The pharmaceutical composition according to claim 1, wherein at least one pharmaceutically acceptable excipient is selected from a group comprising diluents, disintegrants, binders, lubricants and glidants or mixtures thereof.

9. The pharmaceutical composition according to claim 8, wherein diluent is lactose.

10. The pharmaceutical composition according to claim 9, wherein the amount of lactose is between 1.00 - 90.00 %, preferably 5.00 - 60.0 % and more preferably it is 5.00 -30.00 % by weight of total formulation.

11. The pharmaceutical composition according to claim 7, in the form of a tablet comprising;
a) 10.0 - 50.0 % flurbiprofen,
b) 5.0 - 30.0 % tramadol HCl,
c) 1.0 - 90.0% lactose,
d) 5 - 45.0 % microcrystalline cellulose,
e) 0.5 - 15.0 % hydroxypropyl cellulose,
f) 0.5 - 15.0% croscarmellose sodium,
g) 0.5 - 5.0% colloidal silicon dioxide,
h) 0.1 - 5.0 % magnesium stearate by weight of total formulation.

12. The pharmaceutical composition according to claim 7, in the form of a bilayer tablet comprising;
• flurbiprofen layer:
a) 10.0 - 50.0 % flurbiprofen,
b) 1.0 - 90.0 % lactose,
c) 5.0 - 45.0 % microcrystalline cellulose,
d) 0.5 - 15.0 % croscarmellose sodium,
e) 0.5 - 15.0 % hydroxypropyl cellulose,
f) 0.3 - 5.0% colloidal silicon dioxide,
g) 0.1 - 5.0 % magnesium stearate
• tramadol HCl layer:
h) 5.0 - 30 % tramadol HCl,
i) 8.0 - 45.0 % microcrystalline cellulose,
j) 1.0 - 90.0 % lactose,
k) 0.5 - 15 % sodium starch glycolate,
l) 0.5 - 15.0% hydroxypropylcellulose,
m) 0.1 - 5.0% colloidal silicon dioxide,
n) 0.1 -5.0% magnesium stearate by weight of total formulation.

13. The pharmaceutical composition according to claim 7, in the form of a trilayer tablet comprising;
• flurbiprofen layer:
a) 10.0 - 50% flurbiprofen,
b) 1.0 - 90.0 % lactose,
c) 8.0 - 45.0 % microcrystalline cellulose,
d) 0.5 - 15.0 % croscarmellose sodium,
e) 0.5 - 15.0 % hydroxypropyl cellulose,
f) 0.3 - 5.0% colloidal silicon dioxide,
g) 0.1 - 5.0 % magnesium stearate
• interlayer:
h) 0.10 - 5.0 % magnesium starate,
i) 8.0 - 90.0 % microcrystalline cellulose,
j) 0.5 - 20.0 % hydroxypropyl-cellulose,
k) 0.01 - 1.0 % iron oxide yellow.
l) 0.3 - 5.0% colloidal silicon dioxide,
• tramadol HCl layer:
m) 5.0 - 30.0 % tramadol HCl,
n) 1.0 - 90.0 % lactose,
o) 0.5 - 15.0 % sodium starch glycolate,
p) 0.5 - 15.0 % hydroxypropylcellulose,
q) 0.1 - 5.0 % colloidal silicon dioxide,
r) 0.1 - 5.0 % magnesium stearate by weight of total formulation.
